# FASCICULE DE BREVET EUROPEEN

(11) **EP 2 220 256 B1**
(45) Date de publication et mention de la délivrance du brevet: **01.01.2014**
(21) Numéro de dépôt: 08851071.4
(22) Date de dépôt: 21.11.2008
(51) Int. Cl.: C12Q 1/68

(54) **PROCÉDÉ IN VITRO DE DIAGNOSTIC DU CANCER DE LA PEAU**
IN-VITRO-VERFAHREN ZUR DIAGNOSE VON HAUTKREBS
IN VITRO METHOD FOR DIAGNOSING SKIN CANCER

(30) Priorité: 21.11.2007 FR 0759193
(43) Date de publication de la demande: 25.08.2010
(73) Titulaire: Assistance Publique - Hôpitaux de Paris, 75004 Paris (FR)
(72) Inventeur: SOUFIR, Nadem, F-92140 Clamart (FR)
(74) Mandataire: Regimbeau
(86) Numéro de dépôt international: PCT/EP2008/066028
(87) Numéro de publication internationale: WO 2009/065944

(56) Documents cités:
- RUNDSHAGEN UTA ET AL: "Mutations in the MATP gene in five German patients affected by oculocutaneous albinism type 4." HUMAN MUTATION, vol. 23, no. 2, février 2004 (2004-02), pages 106-110, XP002486098 ISSN: 1059-7794
- NEWTON J M ET AL: "Mutations in the human orthologue of the mouse underwhite gene (uw) underlie a new form of oculocutaneous albinism, OCA4" AMERICAN JOURNAL OF HUMAN GENETICS, vol. 69, no. 5, novembre 2001 (2001-11), pages 981-988, XP002488065 ISSN: 0002-9297
- HARADA MAMORU ET AL: "Use of an in vitro immunoselected tumor line to identify shared melanoma antigens recognized by HLA-A*0201-restricted T cells" CANCER RESEARCH, vol. 61, no. 3, 1 février 2001 (2001-02-01), pages 1089-1094, XP002486099 ISSN: 0008-5472
- GERSTENBLITH MEG R ET AL: "Comprehensive evaluation of allele frequency differences of MC1R variants across populations" HUMAN MUTATION, vol. 28, no. 5, mai 2007 (2007-05), pages 495-505, XP002520648 ISSN: 1059-7794
- NORTON HEATHER L ET AL: "Genetic evidence for the convergent evolution of light skin in Europeans and East Asians." MOLECULAR BIOLOGY AND EVOLUTION MAR 2007, vol. 24, no. 3, mars 2007 (2007-03), pages 710-722, XP002486100 ISSN: 0737-4038
- DU JINYAN ET AL: "Identification of Aim-1 as the underwhite mouse mutant and its transcriptional regulation by MITF" JOURNAL OF BIOLOGICAL CHEMISTRY, vol. 277, no. 1, 4 janvier 2002 (2002-01-04), pages 402-406, XP002486101 ISSN: 0021-9258
- FERNANDEZ L.P. ET AL.: "SLC45A2: a novel malignant melanoma-associated gene" HUMAN MUTATION, vol. 0, 18 juin 2008 (2008-06-18), pages 1-7, XP002486102
- GUEDJ MICKAEL ET AL: "Variants of the MATP/SLC45A2 gene are protective for melanoma in the French population" HUMAN MUTATION, vol. 29, no. 9, septembre 2008 (2008-09), pages 1154-1160, XP002520649 ISSN: 1059-7794
- DATABASE EMBASE [en ligne] ELSEVIER SCIENCE PUBLISHERS, AMSTERDAM, NL Février 2010 DUFFY D L ET AL: 'Multiple pigmentation gene polymorphisms account for a substantial proportion of risk of cutaneous malignant melanoma' Database accession no. EMB-2010076636 & DUFFY D L ET AL: "Multiple pigmentation gene polymorphisms account for a substantial proportion of risk of cutaneous malignant melanoma", JOURNAL OF INVESTIGATIVE DERMATOLOGY 2010 NATURE PUBLISHING GROUP USA LNKD- DOI:10.1038/JID.2009.258, vol. 130, no. 2, February 2010 (2010-02), pages 520-528, ISSN: 0022-202X

### SEQUENCE LISTING

<110> Assistance publique- Hopitaux de Paris SOUFIR, Nadem
<120> Procede in vitro de diagnostic du cancer de la peau
<130> 353788
<150> FR 07/59193
   <151> 2007-11-21
<160> 10
<170> PatentIn version 3.3
<210> 1
   <211> 40060
   <212> DNA
   <213> Homo sapiens
<400> 1
<210> 2
   <211> 601
   <212> DNA
   <213> Homo sapiens
<220>
   <221> misc_feature
   <222> (301)..(301)
   <223> N= C ou G
<400> 2
   ttccctttca ttttccagag aaacttgatc aggaacccac tgattccaag agcaaagtaa      60
   tcagtgagga aatgacacct agaattcatg atgaaaaaag gatgctttat atggtccttt     120
   ttaaggtgat agtttttcct gacgtccata gatttattaa gaatctggta ttttaaacag     180
   taggaaatac acatagaaat atcaaatcca agttgtgcta gaccagaaac ttttagaaga     240
   catccttagg agagagaaag acttacaaga ataaagtgag gaaaacacgg agttgatgca     300
   naagccccaa catccaacct cgactcctct ttcgtagatg agaaactctg tggagttgtg     360
   tgcactatag ggatccccgc ggtacacaat ctgaaagaga gattggaggc tgttgaggta     420
   caaatgcaat gtagtaagaa cccttctcat gcactctgct tctccacctc tggggagcaa     480
   atgtccctgc ctgagggacc ctttctagag tacagagctg atgctgtcat gaccaagtcg     540
   catcctatca catcttcatt caaggaccta tcagaaatct aatgcttgct gcatcagatc     600
   t                                                                     601
<210> 3
   <211> 460
   <212> PRT
   <213> Homo sapiens
<400> 3
   Met Gly Ser Asn Ser Gly Gln Ala Gly Arg His Ile Tyr Lys Ser Leu
   1               5                   10                  15
   Ala Asp Asp Gly Pro Phe Asp Ser Val Glu Pro Pro Lys Arg Pro Thr
               20                  25                  30
   Ser Arg Leu Ile Met His Ser Met Ala Met Phe Gly Arg Glu Phe Cys
           35                  40                  45
   Tyr Ala Val Glu Ala Ala Tyr Val Thr Pro Val Leu Leu Ser Val Gly
       50                  55                  60
   Leu Pro Ser Ser Leu Tyr Ser Ile Val Trp Phe Leu Ser Pro Ile Leu
   65                  70                  75                  80
   Gly Phe Leu Leu Gln Pro Val Val Gly Ser Ala Ser Asp His Cys Arg
                   85                  90                  95
   Ser Arg Trp Gly Arg Arg Arg Pro Tyr Ile Leu Thr Leu Gly Val Met
               100                 105                 110
   Met Leu Val Gly Met Ala Leu Tyr Leu Asn Gly Ala Thr Val Val Ala
           115                 120                 125
   Ala Leu Ile Ala Asn Pro Arg Arg Lys Leu Val Trp Ala Ile Ser Val
       130                 135                 140
   Thr Met Ile Gly Val Val Leu Phe Asp Phe Ala Ala Asp Phe Ile Asp
   145                 150                 155                 160
   Gly Pro Ile Lys Ala Tyr Leu Phe Asp Val Cys Ser His Gln Asp Lys
                   165                 170                 175
   Glu Lys Gly Leu His Tyr His Ala Leu Phe Thr Gly Phe Gly Gly Ala
               180                 185                 190
   Leu Gly Tyr Leu Leu Gly Ala Ile Asp Trp Ala His Leu Glu Leu Gly
           195                 200                 205
   Arg Leu Leu Gly Thr Glu Phe Gln Val Met Phe Phe Phe Ser Ala Leu
       210                 215                 220
   Val Leu Thr Leu Cys Phe Thr Val His Leu Cys Ser Ile Ser Glu Ala
   225                 230                 235                     240
   Pro Leu Thr Glu Val Ala Lys Gly Ile Pro Pro Gln Gln Thr Pro Gln
                   245                 250                 255
   Asp Pro Pro Leu Ser Ser Asp Gly Met Tyr Glu Tyr Gly Ser Ile Glu
               260                 265                 270
   Lys Val Lys Asn Gly Tyr Val Asn Pro Glu Leu Ala Met Gln Gly Ala
           275                 280                 285
   Lys Asn Lys Asn His Ala Glu Gln Thr Arg Arg Ala Met Thr Leu Lys
       290                 295                 300
   Ser Leu Leu Arg Ala Leu Val Asn Met Pro Pro His Tyr Arg Tyr Leu
   305                 310                 315                     320
   Cys Ile Ser His Leu Ile Gly Trp Thr Ala Phe Leu Ser Asn Met Leu
                   325                 330                 335
   Phe Phe Thr Asp Phe Met Gly Gln Ile Val Tyr Arg Gly Asp Pro Tyr
               340                 345                 350
   Ser Ala His Asn Ser Thr Glu Phe Leu Ile Tyr Glu Arg Gly Val Glu
           355                 360                 365
   Val Gly Cys Trp Gly Phe Cys Ile Asn Ser Val Phe Ser Ser Leu Tyr
       370                 375                 380
   Ser Tyr Phe Gln Lys Val Leu Val Ser Tyr Ile Gly Leu Lys Gly Leu
   385                 390                 395                     400
   Tyr Phe Thr Gly Tyr Leu Leu Phe Gly Leu Gly Thr Gly Phe Ile Gly
                   405                 410                 415
   Leu Phe Pro Asn Val Tyr Ser Thr Leu Val Leu Cys Ser Leu Phe Gly
               420                 425                 430
   Val Met Ser Ser Thr Leu Tyr Thr Val Pro Phe Asn Leu Ile Thr Glu
           435                 440                 445
   Tyr His Arg Glu Glu Glu Lys Glu Val Cys Cys His
       450                 455                 460
<210> 4
   <211> 601
   <212> DNA
   <213> Homo sapiens
<220>
   <221> misc_feature
   <222> (301)..(301)
   <223> N= C ou T
<400> 4
   tgctctgaga ggagtaagag acaggaggac cagaggaaaa tgcagacatt tttctttgag      60
   atataaaatt ttaattccaa gggatgatag ctacggggga tttgggaatt cagttagacc     120
   ccatgaaact cttctcgtca aacagacaaa acaaacaatt aaaaaaacaa caacaacaaa     180
   gagcaagaat attttccctt gtaaagaaaa aatgttgcat ctttacctgt tcagcatgat     240
   ttttgttttt tgctccctgc attgccagct ctggatttac gtaaccattt ttaactttct     300
   ngatagaacc atactcgtac attccatctg atgacaatgg agggtcctga ggggtttgct     360
   gtgggggaat gccctttgca acctctgtaa gtggggcttc agagatactg cacagatgaa     420
   cagtaaaaca caaagtgagc accaatgcag agaagaagaa catgacctgg aattctgtac     480
   ccaacagtct tcccagctcc agatgggccc agtctatagc acccaaaagg taacccaggg     540
   cacctccaaa acctggaaag caagaaaagc tatgttagca tatttagcaa attatcgttc     600
   a                                                                     601
<210> 5
   <211> 317
   <212> PRT
   <213> homo sapiens
<400> 5
   Met Ala Val Gln Gly Ser Gln Arg Arg Leu Leu Gly Ser Leu Asn Ser
   1               5                   10                  15
   Thr Pro Thr Ala Ile Pro Gln Leu Gly Leu Ala Ala Asn Gln Thr Gly
               20                  25                  30
   Ala Arg Cys Leu Glu Val Ser Ile Ser Asp Gly Leu Phe Leu Ser Leu
           35                  40                  45
   Gly Leu Val Ser Leu Val Glu Asn Ala Leu Val Val Ala Thr Ile Ala
       50                  55                  60
   Lys Asn Arg Asn Leu His Ser Pro Met Tyr Cys Phe Ile Cys Cys Leu
   65                  70                  75                  80
   Ala Leu Ser Asp Leu Leu Val Ser Gly Ser Asn Val Leu Glu Thr Ala
                   85                  90                  95
   Val Ile Leu Leu Leu Glu Ala Gly Ala Leu Val Ala Arg Ala Ala Val
               100                 105                 110
   Leu Gln Gln Leu Asp Asn Val Ile Asp Val Ile Thr Cys Ser Ser Met
   115 120 125
   Leu Ser Ser Leu Cys Phe Leu Gly Ala Ile Ala Val Asp Arg Tyr Ile
   130 135 140
   Ser Ile Phe Tyr Ala Leu Arg Tyr His Ser Ile Val Thr Leu Pro Arg
   145 150 155 160
   Ala Arg Arg Ala Val Ala Ala Ile Trp Val Ala Ser Val Val Phe Ser
   165 170 175
   Thr Leu Phe Ile Ala Tyr Tyr Asp His Val Ala Val Leu Leu Cys Leu
   180 185 190
   Val Val Phe Phe Leu Ala Met Leu Val Leu Met Ala Val Leu Tyr Val
   195 200 205
   His Met Leu Ala Arg Ala Cys Gln His Ala Gln Gly Ile Ala Arg Leu
   210 215 220
   His Lys Arg Gln Arg Pro Val His Gln Gly Phe Gly Leu Lys Gly Ala
   225 230 235 240
   Val Thr Leu Thr Ile Leu Leu Gly Ile Phe Phe Leu Cys Trp Gly Pro
   245 250 255
   Phe Phe Leu His Leu Thr Leu Ile Val Leu Cys Pro Glu His Pro Thr
   260 265 270
   Cys Gly Cys Ile Phe Lys Asn Phe Asn Leu Phe Leu Ala Leu Ile Ile
   275 280 285
   Cys Asn Ala Ile Ile Asp Pro Leu Ile Tyr Ala Phe His Ser Gln Glu
   290 295 300
   Leu Arg Arg Thr Leu Lys Glu Val Leu Thr Cys Ser Trp
   305 310 315
<210> 6
   <211> 51
   <212> DNA
   <213> homo sapiens
<220>
   <221> misc_feature
   <222> (26)..(26)
   <223> N = A or C
<400> 6
   tcatctgctg cctggccttg tcgganctgc tggtgagcgg gagcaacgtg c 51
<210> 7
   <211> 601
   <212> DNA
   <213> homo sapiens
<220>
   <221> misc_feature
   <222> (301)..(301)
   <223> N = T or G
<400> 7
   gtgagcttgg tggagaacgc gctggtggtg gccaccatcg ccaagaaccg gaacctgcac     60
   tcacccatgt actgcttcat ctgctgcctg gccttgtcgg acctgctggt gagcgggagc    120
   aacgtgctgg agacggccgt catcctcctg ctggaggccg gtgcactggt ggcccgggct    180
   gcggtgctgc agcagctgga caatgtcatt gacgtgatca cctgcagctc catgctgtcc    240
   agcctctgct tcctgggcgc catcgccgtg gaccgctaca tctccatctt ctacgcactg    300
   ngctaccaca gcatcgtgac cctgccgcgg gcgcggcgag ccgttgcggc catctgggtg    360
   gccagtgtcg tcttcagcac gctcttcatc gcctactacg accacgtggc cgtcctgctg    420
   tgcctcgtgg tcttcttcct ggctatgctg gtgctcatgg ccgtgctgta cgtccacatg    480
   ctggcccggg cctgccagca cgcccagggc atcgcccggc tccacaagag gcagcgcccg    540
   gtccaccagg gctttggcct taaaggcgct gtcaccctca ccatcctgct gggcattttc    600
   t                                                                    601
<210> 8
   <211> 601
   <212> DNA
   <213> homo sapiens
<220>
   <221> misc_feature
   <222> (301)..(301)
   <223> N = C or T
<400> 8
   gtggccacca tcgccaagaa ccggaacctg cactcaccca tgtactgctt catctgctgc 60
   ctggccttgt cggacctgct ggtgagcggg agcaacgtgc tggagacggc cgtcatcctc 120
   ctgctggagg ccggtgcact ggtggcccgg gctgcggtgc tgcagcagct ggacaatgtc 180
   attgacgtga tcacctgcag ctccatgctg tccagcctct gcttcctggg cgccatcgcc 240
   gtggaccgct acatctccat cttctacgca ctgcgctacc acagcatcgt gaccctgccg 300
   ngggcgcggc gagccgttgc ggccatctgg gtggccagtg tcgtcttcag cacgctcttc 360
   atcgcctact acgaccacgt ggccgtcctg ctgtgcctcg tggtcttctt cctggctatg 420
   ctggtgctca tggccgtgct gtacgtccac atgctggccc gggcctgcca gcacgcccag 480
   ggcatcgccc ggctccacaa gaggcagcgc ccggtccacc agggctttgg ccttaaaggc 540
   gctgtcaccc tcaccatcct gctgggcatt ttcttcctct gctggggccc cttcttcctg 600
   c 601
<210> 9
   <211> 51
   <212> DNA
   <213> homo sapiens
<220>
   <221> misc_feature
   <222> (26)..(26)
   <223> N = G or C
<400> 9
   cctcatcatc tgcaatgcca tcatcnaccc cctcatctac gccttccaca g 51
<210> 10
   <211> 3115
   <212> DNA
   <213> homo sapiens
<400> 10
   agacgcagtc ttcagcaagg aagtgctggg aacgccctgg agtgaaccca ggaagatgcc 60
   tgcagtgggt gccagggccc ctctccaccg tccctgctgg gcttcggggc cacgcccgac 120
   tgctgtgaac ggcctgcgga gcaccacgtg cgacggctgg aggcgagagg tctgcctttg 180
   atgtggctgt tggtgcaggg cctgtggtgc cttccgcagc ggaaatggcg cgccgcccgg 240
   ggagggcggg agcagcgtcc cgggtgcccc tgtgaggatg agcgacgaga tgactggagg 300
   gtccctgaag acctcactag ggtgccccca gccggtccgc tcccaggaag cgacaccccc 360
   acagccccag ggctgcagct gagggggtcg ccactctggc tgggcgaggc tgggcccttg 420
   ggggcaggcg ccagagtggc ctcaggctct acaagatgcc tgaaaacacc aacctctcca 480
   gggctcacta gcattggacg ctttcacgct ctgccctggc cggaagcccc ctcaccccgc 540
   gcgatgtgca aactcctgca gggctcactc agtttccaga actttaatta ttggaaagtt 600
   ctccctggtc cagcccccaa atctgccgtg aacgttgaca gctgagttgc tgctccatgc 660
   gtgctttggc tgagagcaga ggggacccct gtcctccctg agctgctgac gaggggaggg 720
   gtgaagggtg gggcctctgg agagggcagg tcccggggaa gctccggact cctagagggg 780
   cggccaggtg ggggccctgg tgaccaggac agactgtggt gttttttaac gtaaaggaga 840
   tccgcggtgt gagggacccc ctgggtcctg cacgccgcct ggtggcaggc cgggccatgg 900
   tgggtgctca cgcccccggc atgtggccgc cctcagtggg aggggctctg agaacgactt 960
   tttaaaacgc agagaaaagc tccattcttc ccaggacctc agcgcagccc tggcccagga 1020
   aggcaggaga cagaggccag gacggtccag aggtgtcgaa atgtcctggg gacctgagca 1080
   gcagccacca gggaagaggc agggagggag ctgaggacca ggcttggttg tgagaatccc 1140
   tgagcccagg cggtagatgc caggaggtgt ctggactggc tgggccatgc ctgggctgac 1200
   ctgtccagcc agggagaggg tgtgagggca gatctggggg tgcccagatg gaaggaggca 1260
   ggcatggggg acacccaagg ccccctggca gcaccatgaa ctaagcagga cacctggagg 1320
   ggaagaactg tggggacctg gaggcctcca acgactcctt cctgcttcct ggacaggact 1380
   atggctgtgc agggatccca gagaagactt ctgggctccc tcaactccac ccccacagcc 1440
   atcccccagc tggggctggc tgccaaccag acaggagccc ggtgcctgga ggtgtccatc 1500
   tctgacgggc tcttcctcag cctggggctg gtgagcttgg tggagaacgc gctggtggtg   1560
   gccaccatcg ccaagaaccg gaacctgcac tcacccatgt actgcttcat ctgctgcctg   1620
   gccttgtcgg acctgctggt gagcgggagc aacgtgctgg agacggccgt catcctcctg   1680
   ctggaggccg gtgcactggt ggcccgggct gcggtgctgc agcagctgga caatgtcatt   1740
   gacgtgatca cctgcagctc catgctgtcc agcctctgct tcctgggcgc catcgccgtg   1800
   gaccgctaca tctccatctt ctacgcactg cgctaccaca gcatcgtgac cctgccgcgg   1860
   gcgcggcgag ccgttgcggc catctgggtg gccagtgtcg tcttcagcac gctcttcatc   1920
   gcctactacg accacgtggc cgtcctgctg tgcctcgtgg tcttcttcct ggctatgctg   1980
   gtgctcatgg ccgtgctgta cgtccacatg ctggcccggg cctgccagca cgcccagggc   2040
   atcgcccggc tccacaagag gcagcgcccg gtccaccagg gctttggcct taaaggcgct   2100
   gtcaccctca ccatcctgct gggcattttc ttcctctgct ggggcccctt cttcctgcat   2160
   ctcacactca tcgtcctctg ccccgagcac cccacgtgcg gctgcatctt caagaacttc   2220
   aacctctttc tcgccctcat catctgcaat gccatcatcg accccctcat ctacgccttc   2280
   cacagccagg agctccgcag gacgctcaag gaggtgctga catgctcctg gtgagcgcgg   2340
   tgcacgcggc tttaagtgtg ctgggcagag ggaggtggtg atattgtgtg gtctggttcc   2400
   tgtgtgaccc tgggcagttc cttacctccc tggtccccgt ttgtcaaaga ggatggacta   2460
   aatgatctct gaaagtgttg aagcgcggac ccttctgggt ccagggaggg gtccctgcaa   2520
   aactccaggc aggacttctc accagcagtc gtggggaacg gaggaggaca tggggaggtt   2580
   gtggggcctc aggctccggg caccaggggc caacctcagg ctcctaaaga gacattttcc   2640
   gcccactcct gggacactcc gtctgctcca atgactgagc agcatccacc ccaccccatc   2700
   tttgctgcca gctctcagga ccgtgccctc gtcagctggg atgtgaagtc tctgggtgga   2760
   agtgtgtgcc aagagctact cccacagcag ccccaggaga aggggctttg tgaccagaaa   2820
   gcttcatcca cagccttgca gcggctcctg caaaaggagg tgaaatccct gcctcaggcc   2880
   aagggaccag gtttgcagga gcccccctag tggtatgggg ctgagccctc ctgagggccg   2940
   gttctaaggc tcagactggg cactggggcc tcagcctgct ttcctgcagc agtcgcccaa   3000
   gcagacagcc ctggcaaatg cctgactcag tgaccagtgc ctgtgagcat ggggccagga   3060
   aagtctggta ataaatgtga ctcagcatca cccaccttaa aaaaaaaaaa aaaaa        3115

## Description

La présente invention concerne le diagnostic du cancer de la peau et, plus spécifiquement, un procédé de diagnostic *in vitro* du cancer cutané pour un sujet suspecté d'avoir ou d'être prédisposé au cancer cutané.

La connaissance de la prédisposition génétique au mélanome a considérablement évolué ces quinze dernières années. Deux importants gènes de prédisposition au mélanome ont ainsi été identifiés, lesquels gènes correspondent à CDKN2A et CDK4. Ces deux gènes sont essentiellement impliqués dans la prédisposition familiale et dans de multiples cas sporadiques de mélanomes (HUSSUSSIAN et al., Nat. Genet., vol.8, p:15-21, 1994 ;

KAMB et al., Nat. Genet., vol.8, p:23-6, 1994; ZUO et al., Nat. Genet.,vol.12, p:97-9, 1996.).

Des études récentes ont identifiés des loci majeurs impliqués dans la susceptibilité sur les chromosomes 1p22 (GILLANDERS et al., Am. J. Hum. Genet., vol. 73, p:301-13, 2003) et 9q21 (JONSSON et al., J. Natl. Cancer Inst., vol.97, p:1377-82, 2005).

Pour autant, il existe aujourd'hui des évidences récurrentes d'une implication de multiples facteurs génétiques dans la prédisposition au mélanome et l'identification d'autres variants génétiques associés à une moindre contribution au risque de mélanome est en progrès. Parmi ces nouveaux variants, on peut ainsi citer les variants de type RHC du gène *MC1R*, pour lequel il a été montré qu'il constitue un gène du mélanome de faible pénétrance (PALMER et al., Am. J. Hum Genet., vol.66, p:176-86, 2000 ; KENNEDY et al., J. Invest. Dermatol., vol.117, p:294-300, 2001 ; MATICHARD et al., J. Med. Genet., vol.41, p:e13, 2004; STRATIGOS et al., J. Invest. Dermatol., 2006 ; LANDI et al., J Natl. Cancer Inst., vol.97, p:998-1007, 2005 ; DEBNIAK et al., Int. J. Cancer, vol. 119, p:2597-602, 2006 ; HEALY, Photodermatol. Photoimmunol. Photomed., vol.20, p:283-8, 2004) et qu'il agit comme un élément modificateur du risque de mélanome chez les individus portant des mutations de CDKN2A (BOX et al., Am. J. Hum. Genet., vol.69, p:4, 2001; VAN DER VELDEN et al., Am. J. Hum. Genet., vol.69, p:4, 2001; CHAUDRU et al., Cancer Epidemiol. Biomarkers Prev., vol.14, p:2384-90, 2005.). Cinq variants alléliques de *MC1R* (D84E, R142H, R151C, R160W et D294H) ont ainsi été identifiés comme étant fortement associé avec un phénotype caractérisé par des cheveux roux, une peau claire, des tâches de rousseur et la sensibilité au soleil et sont connus maintenant sous le terme variants RHC.

Pour autant, il existe encore un besoin important d'identifier d'autres gènes impliqués dans la susceptibilité au cancer de la peau afin d'identifier clairement et complètement les sujets présentant la plus grande prédisposition au mélanome de sorte de suivre ces sujets en priorité.

La présente invention est basée sur la découverte d'une association forte entre certains allèles du gène *MATP*/*SLC45A2* et le mélanome et, plus spécifiquement, entre l'allèle *SLC45A2* 374F et un risque accru de mélanome, alors que l'allèle *SLC45A2* 374L présente un effet protecteur vis-à-vis du mélanome. En outre, il avait été préalablement démontré que l'allèle *SLC45A2* 374F était associé à une diminution de la quantité de mélanine photoprotectrice (eumélanine) dans l'épiderme (NORTON et al., Mol Biol Evol., 24:710-22 ; 2007), ce qui suggère soit une diminution de l'expression, soit une perte de fonction de la protéine MATP/SLC45A2. A partir des résultats obtenus par les inventeurs, il est donc également possible de conclure que la présence de l'allèle 374F du gène *MATP*/*SLC45A2* est également associée à un risque accru de mélanome, et que la présence de l'allèle *SLC45A2* 374L est associée à un effet protecteur contre le mélanome.

Par « gène *MATP*/*SLC45A2* », on entend ici le gène codant pour la protéine transporteur associée aux membranes (MATP) originellement appelée AIM1, et récemment renommée « membre 2 de la famille des transporteurs de soluté » (ou SLC45A2). Le gène *MATP*/*SLC45A2* est situé sur le chromosome 5p (Gene ID 51151) et présente la séquence telle que décrite au numéro d'accession NT_006576.15 (SEQ ID NO :1). La protéine MATP/SLC45A2 (Séquence de l'ARNm: numéro d'accession NM_001012509) présente la séquence protéique SEQ ID NO:3 (numéro d'accession : NP_001012527).

La protéine MATP/SLC45A2 est désignée dans la présente indifféremment par les termes « MATP », « SLC45A2 » ou « MATP/SLC45A2 ».

Du fait de sa surexpression établie dans certaines lignées cellulaires de mélanome par rapport à son expression dans les mélanocytes normaux (absence d'expression dans les tissus normaux), le gène *MATPlSLC45A2* était connu comme un antigène de mélanome (Harada M et al, Cancer Research, 1089-1094 ; 2001).

La présente invention repose sur le fait que l'allèle *SLC45A2* 374F est associé à un risque accru de mélanome, tandis que l'allèle *SLC45A2* 374L présente au contraire un effet protecteur vis-à-vis du mélanome.

Le polymorphisme SLC45A2 L374F était déjà connu à la date de l'invention. Ce polymorphisme avait en effet été décrit dans l'art antérieur comme l'un des nombreux polymorphismes identifiés chez des individus souffrant d'albinisme occulocutané. Chez de tels patients, ce variant semble assez commun et 8,5% des individus testés présentent ce variant de façon hétérozygote (Rundshagen U. et al, Human mutation ; 23 :106-110 ; 2004). Le parallèle entre ce polymorphisme et la perte de pigmentation liée à l'albinisme n'est cependant pas suggéré. De même, Newton et al (Am, J. Hum. Genetic ; 69: 981-988 ; 2001) avait identifié ce polymorphisme chez 67 individus, y compris chez des individus à la pigmentation normale, et ce de façon homozygote. Selon ce dernier article, la mutation L374F de la protéine SLC45A2 est « neutre » vis-à-vis des mécanismes d'hypo-pigmentation responsables de l'albinisme.

Par ailleurs, d'autres études ont mis en évidence l'association entre l'allèle L374F de *MATP*/*SLC45A2* et la pigmentation humaine normale chez certaines populations, notamment chez les Afro-américains (Norton HL et al, Mol. Biol. Evol, 24(3) :710-722, 2007 ; Graf et al., Hum. Mutat., vol.25, p:278-84, 2005). Cependant, cette association ne saurait suggérer la présente invention, puisque plusieurs centaines de gènes influencent la pigmentation sans pour autant être associés à une prédisposition au mélanome (voir par exemple Sulem P, Nature Genetics ; volume 39, number 12, 2007).

Ainsi, les présents inventeurs ont pu d'ailleurs montrer que, bien qu'il soit associé à la pigmentation, le variant de *MATP*/*SLC45A2* 374 identifié ici prédit le risque de mélanome de façon indépendante des caractéristiques pigmentaires. Plus particulièrement, les présents inventeurs ont montré que l'effet SLC45A2 L374F sur la prédisposition au mélanome persiste même après la stratification sur les caractéristiques de pigmentation ou dans un modèle de régression logistique intégrant les 2 facteurs de risque génétique (MATP L374F, variants MC1R) et les facteurs de risque cliniques (couleur des yeux, des cheveux, phototype et nombre de naevus.) Ceci suggère qu'au regard du risque de mélanome, l'information liée au génotype SLC45A2 L374F n'est pas redondante avec les caractéristiques de pigmentation, et que ce variant est donc un facteur de risque de mélanome fort et indépendant (voir aussi l'article des inventeurs Guedj M et al, Human Mutation 29(9), 1154-1160, 2008).

En conséquence, un premier objet de l'invention porte sur un procédé *in vitro* destiné à identifier un sujet atteint ou présentant une prédisposition au cancer de la peau, caractérisé en ce qu'il comprend l'étape d'analyse d'un échantillon biologique provenant dudit sujet par :
a) la détection d'un polymorphisme du gène *MATP*/*SLC45A2* (SEQ ID NO : 1), associé à un seul nucléotide (SNP) est choisi dans le groupe comprenant le SNP rs 1689182 (nucléotide N à la position 301 de SEQ ID NO: 2, le nucléotide G étant associé à un cancer cutané) et aboutissant à la présence d'une phénylalanine en position 374 de la protéine MATP/SLC45A2 (SEQ ID NO :3) et le SNP rs26722 (nucléotide N à la position 301 de SEQ ID n° 4, le nucléotide C étant associé à un cancer cutané) aboutissant à la présence d'un résidu Glutamate en position 272 de la protéine MATP/SLC45A2 (SEQ ID NO :3).

Tel qu'utilisé ici, le terme « sujet » se réfère à un mammifère, de préférence à un humain.

Tel qu'utilisé ici, le terme « échantillon biologique » se réfère à tout échantillon solide ou liquide issu d'un sujet. A titre d'exemple d'échantillons solides, on peut citer un échantillon de peau et, à titre d'exemple d'échantillons liquides, on peut citer un échantillon de sang.

De préférence, ledit échantillon biologique est un échantillon sanguin lorsque l'on effectue une étape de détection d'un polymorphisme du gène *MATP*/*SLC45A2*.

De préférence encore, ledit échantillon biologique est un échantillon de peau lorsque l'on effectue une étape d'analyse de l'expression du gène *MATP*/*SLC45A2*.

Tel qu'utilisé ici, le terme « cancer de la peau » se réfère à un cancer impliquant un type cellulaire de l'épiderme ou du derme, de préférence ledit cancer de la peau est un mélanome.

Avantageusement encore, ledit polymorphisme du gène *MATPlSLC45A2* est associé à un niveau d'expression significativement plus faible du gène *MATP*/*SLC45A2*.

De préférence, ledit SNP correspond au SNP rs16891982 (nucléotide N à la position 301 de SEQ ID NO : 2, le nucléotide G étant associé à un cancer cutané) et aboutissant à la présence d'une phénylalanine en position 374 de la protéine MATP/SLC45A2 (SEQ ID NO :3).

L'homme du métier au regard de ses connaissances générales et de la présente description pourra identifier simplement d'autres polymorphismes du gène *MATP*/*SLC45A2* associé à une prédisposition au cancer de la peau.

Dans un mode de réalisation préféré, le procédé de l'invention permet d'identifier un sujet atteint ou présentant une prédisposition au cancer de la peau en analysant, outre l'un des polymorphismes du gène *MATP*/*SLC45A2* et/ou son expression, un polymorphisme d'un autre gène de susceptibilité au mélanome.

Cet autre gène de susceptibilité est par exemple le gène *MC1R* (gène codant pour le récepteur à la mélanocortine 1). Les présents inventeurs ont en effet pu montrer que quatre polymorphismes du gène du récepteur à la mélanocortine 1, ou *MC1R* (NP_002377.4, SEQ ID NO 5), sont associés à un risque accru de mélanome.

La protéine MC1R est codée par le gène humain *MC1R* (Gene ID 4157), qui est transcrit en un ARNm de séquence NM_002386.3 (SEQ ID NO :10). Cinq variants alléliques de MC1R (D84E, R142H, R151C, R160W et D294H) ont été identifiés comme étant fortement associés avec un phénotype caractérisé par des cheveux roux, une peau claire, des tâches de rousseur et une sensibilité au soleil. Ces variants alléliques sont connus maintenant sous le terme « variants RHC » (FLANAGAN et al., Hum Mol Genet, vol.9, p:2531-7, 2000 ; DUFFY et al., Hum. Mol. Genet.,vol.13, p:447-61, 2004 ; REES, Am. J. Hum. Genet., vol.75, p:739-51, 2004; Sulem P, Nature Genetics ; volume 39, number 12, 2007).

Ces variants ont déjà été associés avec le risque de mélanome dans de multiples populations (STURM et al., Pigment Cell Res., vol. 16, p:266-72, 2003 ; REES, Annu. Rev. Genet., vol.37, p:67-90, 2003), et ont tous été identifiés comme générant une perte de fonction dans les études fonctionnelles (BEAUMONT et al., Hum. Mol. Genet., vol.14, p:2145-54, 2005; BEAUMONT et al., Hum. Mol. Genet., 2007). Les inventeurs ont montré ici encore que certains de ces SNPs de *MC1R* sont associés à un risque accru de mélanome, et ce de façon indépendante aux polymorphismes de *MATP*/*SLC45A2* identifiés précédemment.

Dans ce mode de réalisation, le procédé de l'invention est un procédé *in vitro* destiné à identifier un sujet atteint ou présentant une prédisposition au cancer de la peau, caractérisé en ce qu'il comprend, en outre, c) la détection d'un polymorphisme du gène *MC1R* (SEQ ID NO 10).

Dans un mode de réalisation encore plus préféré, la détection du polymorphisme du gène *MC1R* associé à une prédisposition au cancer de la peau correspond à plusieurs SNPs choisis de préférence dans le groupe comprenant le SNP rs1805006 (nucléotide N à la position 26 de SEQ ID NO : 6 (ss2425919), le nucléotide A étant associé à un cancer cutané) et aboutissant à la présence d'un Glutamate en position 84 de la protéine MC1R (SEQ ID NO :5), le SNP rs1805007 (nucléotide N à la position 301 de SEQ ID NO :7, le nucléotide T étant associé à un cancer cutané) et aboutissant à la présence d'une cystéine en position 151 de la protéine MC1R (SEQ ID NO :5), le SNP rs1805008 (nucléotide N à la position 301 de SEQ ID NO 8, le nucléotide T étant associé à un cancer cutané) et aboutissant à la présence d'un tryptophane en position 160 de la protéine MC1R (SEQ ID NO :5), le SNP rs1805009 (nucléotide N à la position 26 de SEQ ID NO 9, le nucléotide C étant associé à un cancer cutané) et aboutissant à la présence d'une histidine en position 294 de la protéine MC1R (SEQ ID NO :5).

Des techniques pour identifier un polymorphisme du gène *MATP*/*SLC45A2* ou de *MC1R* sont bien connues de l'homme du métier et incluent notamment le polymorphisme de longueur des fragments de restriction (RFLP), des techniques d'hybridation, les techniques de séquençage de l'ADN, la résistance aux exonucléase, le microséquençage, l'extension en phase solide utilisant des ddNTPs, l'extension en solution en utilisant des ddNTPs, des méthodes de ligature d'oligonucléotides, les méthodes pour détecter les SNP telles que hybridation dynamique allèle spécifique, la réaction de ligature en chaîne (LCR), le mini-séquençage, l'utilisation de puces à ADN ou encore l'hybridation d'oligonucléotides allèle spécifique en complément d'une sonde présentant un simple ou un double marquage et à l'aide de réactions de PCR.

De préférence, la dite technique pour identifier un polymorphisme du gène *MATP*/*SLC45A2* ou de *MC1R* est une technique permettant de détecter un polymorphisme associé à un seul nucléotide (SNP).

L'analyse de l'expression du gène *MATP*/*SLC45A2* peut être réalisée grâce à l'une des nombreuses méthodes bien connues de l'homme du métier et permettant la détection du produit d'expression dudit gène tel que son ARN ou son produit protéique.

Dans un mode de réalisation préféré, l'expression du gène *MATP*/*SLC45A2* est effectuée par analyse de l'expression de transcrits d'ARNm ou de précurseurs d'ARNm, tel qu'un ARN natif, dudit gène. Ladite analyse peut être réalisée en préparant l'ARNm/ADNc de cellules d'un échantillon biologique d'un patient, et hybridation de l'ARNm /ADNc avec un polynucléotide de référence. L'ARNm/ADNc préparé peut être utilisé dans une analyse par hybridation ou amplification qui inclut, sans s'y limiter, les analyses Southem et Northen, les analyses par PCR (« polymerase chain reaction »), telle que la PCR quantitative (TAQMAN) et l'utilisation de sondes (« probes arrays ») telles que les matrices ADN GENECHIP®(AFFYMETRIX).

Avantageusement, l'analyse de l'expression du niveau d'ARNm transcrit à partir d'un gène *MATP*/*SLC45A2* implique un procédé d'amplification des acides nucléiques, comme par exemple la RT-PCR (mode de réalisation expérimental décrit dans le Brevet US 4,683,202), la réaction en chaîne par la ligase (BARANY, Proc. Natl. Acad. Sci. USA, vol.88, p: 189-193, 1991), la réplication de séquences auto-entretenue (« self sustained sequence replication ») (GUATELLI et al., Proc. Natl. Acad. Sci. USA, vol.87, p: 1874-1878, 1990), le système d'amplification transcriptionnelle. (KWOH et al., Proc. Natl. Acad. Sci. USA, vol.86, p: 1173-1177, 1989), la « Q-Beta Replicase » (LIZARDI et al., Biol. Technology, vol.6, p: 1197, 1988), la « rolling circle replication » (U. S. Patent No. 5,854,033) ou toute autre méthode d'amplification d'acides nucléiques, suivie d'une étape de détection des molécules amplifiées par des techniques bien connues de l'homme du métier. Ces modes de détection sont particulièrement utiles pour la détection de molécules d'acides nucléiques en très faibles quantités. Telles qu'utilisées ici, les amorces d'amplifications sont définies comme étant une paire de molécules d'acides nucléiques qui peuvent s'apparier respectivement aux régions 3' et 5' d'un gène de façon spécifique (brin positif et négatif, ou inversement) et encadrent une courte région dudit gène. De manière générale, les amorces d'amplification ont une longueur de 10 à 30 nucléotides et permettent l'amplification d'une région d'une longueur comprise entre 50 et 200 nucléotides.

Dans un autre mode de réalisation préféré, la mesure de l'expression du gène *MATP*/*SLC45A2* est réalisée par analyse de l'expression de la protéine traduite à partir dudit gène. Ladite analyse peut être réalisée en utilisant un anticorps (par exemple, un anticorps radiomarqué, marqué avec un chromophore, un fluorophore, ou une enzyme), un dérivé d'anticorps (par exemple un anticorps conjugué à un substrat ou à une protéine ou un ligand d'une protéine d'un couple ligand/protéine (par exemple biotine-streptavidine)) ou un fragment d'anticorps (par exemple un anticorps à une seule chaîne, un domaine hypervariable d'un anticorps isolé, etc.) qui se lie spécifiquement à la protéine traduite à partir du gène *MATP*/*SLC45A*2.

Lesdites analyses peuvent être réalisées par de nombreuses techniques à la portée de l'homme du métier incluant, sans s'y limiter, les tests immunologiques basés sur l'utilisation d'activité enzymatiques (« enzyme immunoassay » EIA), les tests immunologiques basés sur l'utilisation d'isotopes radioactifs (RIA), l'analyse par Western blot et les tests ELISA (« enzyme linked immunoabsorbant assay »).

A titre d'exemple d'anticorps dirigés contre la protéine MATP/SLC45A2, on peut citer les anticorps disponibles chez ABNOVA CORPORATION ou chez SANTA CRUZ BIOTECHNOLOGY.

Pour autant, des anticorps polyclonaux peuvent aussi être préparés par immunisation d'un animal approprié, telle qu'une souris, un lapin ou une chèvre, avec la protéine MATP/SLC45A2 (Homo Sapiens ; SEQ ID NO :3) ou un fragment de celle-ci. La concentration d'anticorps chez l'animal immunisé peut être suivie au cours du temps par des techniques standard, tel qu'un test ELISA, en utilisant un polypeptide immobilisé. Un certain temps après l'immunisation, par exemple, quand les titres d'anticorps spécifiques sont les plus élevés, les cellules produisant les anticorps peuvent être prélevées de l'animal et être utilisées pour préparer des anticorps monoclonaux (mAc) par les techniques standard, telle que la technique des hybridomes initialement décrite par KOHLER et MILSTEIN (Nature, vol.256, p:495-497, 1975), la technique des hybridomes des cellules B humaines (KOZBOR et al., Immunol., vol.4, p: 72, 1983), la technique des hybridomes EBV (COLE et al., In Monoclonal Antibodies and Cancer Therapy, Alan R. Liss,Inc., p: 77-96, 1985) ou la technique des triomes. La technique pour la production d'hybridomes est bien connue (voir : Current Protocols in Immunology, COLIGAN et al. ed., John Wiley & Sons, New York, 1994). Les hybridomes produisant l'anticorps monoclonal désiré sont détectés par criblage des surnageants de culture d'hybridomes pour les anticorps qui se lient au polypeptide d'intérêt, par exemple, en utilisant un test ELISA standard.

Le procédé selon la présente invention peut en outre comprendre une étape de comparaison du niveau d'expression du gène *MATP*/*SLC45A2* dans l'échantillon biologique dudit sujet avec le niveau d'expression dudit gène dans un échantillon contrôle.

A titre d'échantillon contrôle, on peut notamment utiliser un échantillon biologique provenant d'un sujet sain, à savoir qui n'est pas atteint ni prédisposé au cancer de la peau.

Un niveau d'expression significativement plus faible que le niveau d'expression du même gène dans l'échantillon contrôle indique que le patient est atteint ou prédisposé à être atteint d'un cancer de la peau.

Un « niveau d'expression significativement plus faible du gène *MATP*/*SLC45A2*» se réfère à un niveau d'expression dans un échantillon biologique qui est inférieur à au moins 20% du niveau normal d'expression dudit gène, de préférence inférieur à au moins 50% du niveau normal d'expression dudit gène, et de manière particulièrement préférée inférieur d'au moins 90% au niveau normal d'expression dudit gène.

Le niveau « normal » d'expression du gène est le niveau d'expression dudit gène dans un échantillon contrôle correspondant potentiellement à l'échantillon biologique d'un patient ne présentant pas de cancer de la peau ou, de préférence, à la moyenne du niveau d'expression dudit gène dans différents échantillons contrôle.

Un autre objet de la présente invention porte sur l'utilisation, pour la préparation d'une composition destinée au traitement et /ou à la prévention d'un cancer de la peau chez un sujet, d'un composé qui augmente spécifiquement l'expression du gène *MATP*/*SLC45A2* dans une cellule de la peau.

Par cellule de la peau, on entend une cellule du derme ou de l'épiderme. De préférence, ladite cellule de la peau est un mélanocyte.

De préférence, ledit composé augmentant de façon spécifique l'expression du gène *MATP*/*SLC45A2* est choisi dans le groupe comprenant la protéine MATP/SLC45A2 et ses dérivés, un polynucléotide codant pour une telle protéine ou un vecteur comprenant un tel polynucléotide.

Par protéine MATP/SLC45A2, on entend de préférence la protéine MATP/SLC45A2 de *Homo Sapiens* (NP_001012527 ; SEQ ID NO3).

Par « dérivé », on entend une protéine dont la séquence présente un pourcentage d'identité d'au moins 80 %, par exemple d'au moins 85%, de préférence d'au moins 90%, et de manière particulièrement préférée d'au moins 95% avec la séquence polypeptidique de la de la protéine MATP/SLC45A2.

Par pourcentage d'identité entre deux séquences polypeptidiques, on entend le pourcentage d'acides aminés identiques, entre deux séquences devant être comparées, obtenu avec le meilleur alignement possible desdites séquences. Ce pourcentage est purement statistique et les différences entre les deux séquences sont réparties aléatoirement sur toute la longueur des séquences d'acides aminés. Par meilleur alignement possible ou alignement optimal, on entend l'alignement permettant d'obtenir le pourcentage d'identité le plus élevé. Les comparaisons de séquences entre deux séquences d'acides aminés sont habituellement réalisées en comparant lesdites séquences après que celles-ci aient été alignées selon le meilleur alignement possible ; la comparaison est alors réalisée sur des segments de comparaison de façon à identifier et comparer des régions de similarité. Le meilleur alignement possible pour effectuer une comparaison peut être réalisé en utilisant l'algorithme d'homologie globale développé par SMITH et WATERMAN (Ad. App. Math., vol.2, p:482, 1981), en utilisant l'algorithme d'homologie locale développé par NEDDLEMAN et WUNSCH (J. Mol. Biol., vol.48, p:443, 1970), en utilisant la méthode de similarité développé par PEARSON et LIPMAN (Proc. Natl. Acd. Sci. USA, vol.85, p:2444, 1988), en utilisant des programmes d'ordinateur basés sur de tels algorithmes (GAP, BESTFIT, BLAST P, BLAST N, FASTA, TFASTA, Genetics Computer Group, 575 Science Dr., Madison, WI USA), en utilisant les algorithmes d'alignement multiples MUSCLE (Edgar, Robert C., Nucleic Acids Research, vol. 32, p:1792, 2004). Pour obtenir le meilleur alignement possible, on utilisera de préférence le programme BLAST avec la matrice BLOSUM 62 ou la matrice PAM 30. Le pourcentage d'identité est déterminé en comparant les deux séquences alignées de façon optimale, lesdites séquences pourront comprendre des additions ou des délétions au regard de la séquence de référence de sorte d'obtenir le meilleur alignement possible entre ces deux séquences. Le pourcentage d'identité est calculé en déterminant le nombre de position identique entre les deux séquences, en divisant le nombre obtenu par le nombre total de positions comparées et en multipliant le résultat obtenu par 100 pour obtenir le pourcentage d'identité entre ces deux séquences.

Par polynucléotide, on entend une séquence d'ARN ou d'ADN, de préférence ledit polynucléotide est une séquence d'ADN.

Avantageusement encore, ledit polynucléotide est lié de façon opérationnelle à une séquence d'expression génique dirigeant l'expression dudit polynucléotide dans une cellule eucaryote, de préférence dans une cellule de la peau. Ladite séquence d'expression génique correspond à toute séquence de régulation, telle qu'une séquence promotrice ou une combinaison entre une séquence promotrice et une séquence activatrice facilitant la transcription et la traduction efficace du polypeptide tel que décrit précédemment. Ladite séquence d'expression génique peut correspondre à une séquence promotrice virale ou eucaryote, constitutive ou inductible.

À titre d'exemple de vecteurs comprenant ledit polynucléotide, on peut citer les plasmides, les cosmides et les virus, notamment les adénovirus et les rétrovirus.

Avantageusement, ledit composé augmentant spécifiquement l'expression du gène *MATP*/*SLC45A2* peut être associé à un support pharmaceutiquement acceptable.

A titre d'exemple de support pharmaceutiquement acceptable, la composition peut comprendre des émulsions, des microémulsions, des émulsions huile dans l'eau, des lipides anhydres et des émulsions eau dans l'huile, ou d'autres types d'émulsions.

La composition décrite peut comprendre en outre un ou plusieurs additifs tels que les diluants, les excipients, les stabilisateurs et les conservateurs. De tels additifs sont bien connus de l'homme du métier et sont décrits notamment dans « Ullmann's Encyclopedia of Industrial Chemistry, 6th Ed. » (différents éditeurs, 1989-1998, Marcel Dekker); et dans "Pharmaceutical Dosage Forms and Drug Delivery System"s (ANSEL et al., 1994, WILLIAMS & WILKINS).

Un autre objet décrit porte sur un procédé de sélection *in vitro* d'un composé susceptible d'être utile pour le traitement du cancer de la peau, caractérisé en ce qu'il comprend les étapes de :
a) obtention d'une cellule exprimant le gène *MATP*/*SLC45A2* ;
b) mise en contact de ladite cellule avec au moins un composé à tester;
c) comparaison de l'expression du gène *MATP*/*SLC45A2* entre les étapes a) et b) ;
d) sélection du composé induisant une augmentation du niveau d'expression du gène *MATP*/*SLC45A2* dans la cellule à l'étape b) par rapport au niveau d'expression dudit gène à l'étape a).

Avantageusement, ladite cellule exprimant le gène *MATP*/*SLC45A2* est obtenue à partir d'un sujet présentant un cancer de la peau.

De préférence, ladite cellule présente un niveau d'expression significativement plus faible du gène *MATP*/*SLC45A2*, par comparaison à une cellule prélevée sur un sujet ne présentant pas de cancer de la peau.

De préférence, ladite cellule correspond à une cellule de la peau, et plus préférentiellement une cellule issue ou dérivée d'un mélanome.

Tels qu'utilisé ici, le terme « composé » se réfère à tout type de molécules tels que les polypeptides, les polynucléotides, les sucres, les lipides, ou n'importe quel autre composé chimique.

Les méthodes pour déterminer l'expression du gène *MATP*/*SLC45A2* sont bien connues de l'homme du métier. Par exemple, on peut utiliser les méthodes décrites précédemment.

La présente invention sera maintenant décrite plus en détail à l'aide des exemples qui illustrent l'invention sans en limiter aucunement le cadre.

### EXEMPLES

### Matériels et méthodes

### Etude de la population

1019 patients et 1466 sujets de contrôle, tous d'origine Caucasienne, ont été inclus prospectivement dans l'étude entre 2003 et 2006. Les patients « Mélanome » ont été recrutés entre 2003 et 2006 dans la cohorte mélanome (MELAN-COHORT), une cohorte prospective incluant tous les patients avec un mélanome du Département Dermatologique de tous les Universités affiliées aux hôpitaux de Paris (Bichat, Percy, Ambroise Paré, Henri Mondor, Cochin et les Hôpitaux de Saint Louis). L'étude de population a constitué en des patients âgés de 18 à 80 ans avec des mélanomes malins histologiquement avéré. Les patients n'ont pas été inclus s'ils étaient immuno-déficients (HIV ou transplantation), ou s'ils souffraient de génodermatose prédisposant à un cancer de la peau (albinisme, Syndrome de Gorlin, ou épithéliomatose pigmentaire). Le groupe de contrôle était composé de 1466 personnes caucasiennes, sans aucun antécédent de cancer de la peau.

Le comité d'éthique médical local a approuvé le protocole d'étude. Un consentement éclairé a été obtenu de tous les patients et sujets contrôles inscrits pour l'étude. Après ledit consentement informé, l'ADN génomique a été isolé des *leukocytes du sang périphérique* de tous les participants en utilisant le QIAAMP BLOOD MINI KIT (QIAGEN).

### Collecte des données sur les facteurs de risque du mélanome

Les données sur les caractéristiques de la pigmentation ont été collectées pour tous les patients et 220 sujets contrôles. Une interview personnelle et standardisée, et un examen de la peau sur l'ensemble du corps ont été réalisés par un dermatologue. Le compte-rendu des résultats a utilisé un formulaire de rapport d'examen standard.

Les caractéristiques de la peau ont été mesurées par type de peau selon la classification de FISTZPATRICK (FISTZPATRICK, Arch. Dermatol., vol. 124, p:869-71, 1988) et évaluées comme suit : toujours brûlée jamais brunie (type de peau I), toujours brûlée puis brunie (type de peau II) ; toujours brunie parfois brûlé (type de peau III) ; et toujours brunie jamais brûlée (type de peau IV). La couleur des yeux a été classée en tant que foncée (marron ou noire) ou claire (bleu, vert/noisette ou gris) et la couleur originelle des cheveux (avant le gris) a été classée en utilisant 5 catégories : rousse, blonde, claire ou foncée-marron et noire. Le décompte total des naevus du corps (divisé en 4 catégories : <10, 10-50, 50-100, >100), la présence ou l'absence de syndrome de mole atypique (SLADE et al., J Am. Acad. Dermatol., vol.32, p:479-94, 1995) et la présence ou l'absence de lentigines solaires ont aussi été évalués au moyen d'un examen physique. Par ailleurs, dans le groupe de mélanome, la localisation anatomique des mélanomes, l'âge du patient au diagnostic et des données histopathologiques pertinentes ont aussi été rapportés.

### Genotype MC1R, SLC45A2 and OCA2 variables

Les polymorphismes de *MC1R* retenus pour l'analyse génétique étaient ceux associés avec le phénotype couleur de cheveux rousse (Allèles RHC) et incluent c.252 C>A p.D84E, c425G>A p.R142H, c.p.R151C, c.476 C>T p.R160W et c.880G>C p.D294H (FLANAGAN et al., Hum Mol Genet, vol.9, p:2531-7, 2000 ; DUFFY et al., Hum. Mol. Genet.,vol.13, p:447-61, 2004 ; REES, Am. J. Hum. Genet., vol.75, p:739-51, 2004). Ces variants ont aussi été associés avec le risque de mélanome dans de multiples populations (STURM et al., Pigment Cell Res., vol.16, p:266-72, 2003 ; REES, Annu. Rev. Genet., vol.37, p:67-90, 2003), et ont tous été identifiés comme générant une perte de fonction dans les études fonctionnelles (BEAUMONT et al., Hum. Mol. Genet., vol.14, p:2145-54, 2005; BEAUMONT et al., Hum. Mol. Genet., 2007). Les 2 variants de *SLC45A2* étudiés étaient des SNPs non-synonymes (C.1122 C>G, L374F et c.814G>A, E372K) qui avaient été précédemment identifiés comme étant associés avec la pigmentation humaine normale (GRAF et al., Hum. Mutat., vol.25, p:278-84, 2005). Les variants d'*OCA2* étudiés étaient les 3 SNPs introniques récemment présentés pour être fortement associés avec la couleur des yeux, des cheveux, et de la pigmentation de la peau (rs7495174, rs4778241 et rs4778138) dans une population anglo-celtique du Queensland. (DUFFY et al., Am. J. Hum. Genet., vol.80, p:241-52, 2007.)

Tous les SNPs ont été genotypés en utilisant le système de génotypage de SNPs par PCR de KBIOSCIENCE. le système de genotypage de SNPs par PCR de KBIOSCIENCE est un nouveau système homogène et fluorescent de génotypage, utilisant une forme unique de PCR allèle spécifique qui est distincte et différente de la méthode de discrimination allélique conventionnelle. Cette méthode de génotypage a été ensuite validée par une méthode de génotypage indépendante, la méthode de génotypage de discrimination allélique par test TAQMAN des SNPs (APPLIED BIOSYSTEMS). De plus, pour les 2/3 des SNPs, la vérification des génotypes correspondants a aussi été effectuée par le séquençage de 50-100 échantillons d'ADN (ABIPRISM 3130)

Le génotypage des variants de *MC1R* a été effectué avec succès chez 828 patients (82%) et 1067 contrôles (72,78%). Les 2 variants *SLC45A2* ont pu être génotypés efficacement chez 95% des patients et des contrôles. Le génotypage des variants *OCA2* a aussi pu être effectué pour 95% des patients et des contrôles, excepté pour OCA2-rs477824 qui n'a pu être génotypé que sur seulement 81,2% des patients.

### Analyse statistique

L'élément principal de l'analyse statistique a été réalisé en utilisant le COMPUTER R PACKAGE (version 2.4.1). Le niveau de signifiance pour tous les tests a été fixé à un niveau correspondant à un taux d'erreur de type I de α = 5% . Prenant en considération le nombre de tests réalisés et le niveau de leurs associations, la plupart d'entre eux seraient restés significatifs si on avait appliqué une correction de BONFERRONI pour prendre le problème de tests multiples en considération. Tous les rapports odds (ORs) ont été reportés avec leur intervalle de confidence de 95% (CI).

### Association des facteurs génétiques avec le mélanome

La conformité à l'équilibre HARDY-WEINBERG a été testée dans les contrôles en utilisant un classique un degré de liberté, le test chi au carré. L'analyse de l'association individuelle des facteurs génétiques avec les mélanomes (les variables MC1R, SLC45A2 and OCA2) ont été réalisés en comparant les cas et les contrôles en utilisant un test exact de FISHER de génotypes (Codé avec 0, 1 et 2, avec 0 étant le plus fréquent génotype). Les ORs correspondants ont été fixés en utilisant une analyse logique standard de régression sur les données avec, pour chaque polymorphisme, la référence prise comme étant le plus fréquent génotype (0). De plus, une analyse de la diversité haplotypique a été réalisée sur chaque gène par l'algorithme de maximisation de l'attente (EM) en utilisant le logiciel ARLEQUIN (Version 3.01) (SCHNEIDER et al., Genetics and Biometry Lab, Dept of Anthropology, University of Geneva 2000). Le déséquilibre de liaison (LD) entre les paires de sites polymorphiques a été mesurée dans les contrôles en utilisant les statistiques communes D, D' (LEWONTIN, Genetics, vol.49, p:49-67,1964) et r (HILL & ROBERTSON, Genetics, vol.60, p:615-28, 1968). Finalement, les interactions gène-gène ont été évaluées en utilisant le même système de régression logique.

### Association des facteurs cliniques avec les mélanomes

Les analyses individuelles des facteurs cliniques usuels (couleur de la peau : pâle et intermédiaire *versus* foncée, type de peau : I-II *versus* III-IV, couleur des cheveux : rousse-blond-clair marron *versus* foncé marron-noir, couleur des yeux : clair/pâle *versus* noir, le nombre de naevus: <50 *versus* >50, les éphélides et lentigines dorsales : présence *versus* absence ont été aussi réalisées en comparant tous les cas et 220 contrôles avec un test exact de génotypes de FISHER. Les ORs correspondants ont été encore une fois fixés en utilisant une analyse de régression logique sur les données.

### Association des facteurs génétiques avec les facteurs cliniques

Dans la mesure où les trois gènes analysés interviennent dans la pigmentation, les analyses d'association individuelle des facteurs génétiques pour chaque facteur clinique ont aussi été réalisées, adaptées au statut contrôle-cas, de sorte qu'aucune association observée n'était due à l'association entre le mélanome et les facteurs cliniques considérés. Les ORs correspondants ont été examinés via les régressions logiques.

### Intégration de la génétique et des facteurs cliniques

Parce qu'ils ont été associés avec les 3 gènes et avec le mélanome, les facteurs cliniques peuvent créer des confusions potentielles quand on analyse l'association de ces gènes avec la maladie. Dans cette partie des analyses, nous avons utilisé un modèle logique basé sur un groupe de facteurs cliniques et génétiques pour vérifier si les associations génétiques avec le mélanome se révélaient encore en présence des facteurs cliniques. Finalement, les ORs ont été recalculés pour les facteurs génétiques et cliniques finalement sélectionnés comme étant les plus pertinents pour le mélanome.

### Résultats

### L'association des facteurs génétiques avec le mélanome

Pour les contrôles, les fréquences de génotype pour tous les polymorphismes testés se situent dans l'équilibre de HARDY-WEINBERG.

Les résultats pour MC 1 R sont présentés dans le tableau I.

**Tableau I**

| **MC1R genotype** | **Cas** | **Contrôles** | **P** | **OR (Cl)** |
|---|---|---|---|---|
| 0/0 | 510 | 841 | | référence |
| 1/0 | 280 | 202 | < 2,2 x 10⁻¹⁶ | 2,29 (1,85-2,82) |
| 1/1 | 48 | 24 | | 3,3 (2-5,45) |

| **allèles RHC de MC1R** | **Cas** | **Contrôles** | | **OR (Cl)** |
|---|---|---|---|---|
| Pas d'allèles RHC | 0,793 | 0,887 | | référence |
| R151C | 0,088 | 0,0042 | | 2,35 (1,78-3,11) |
| R160W | 0,065 | 0,038 | | 1,88 (1,40-2,54) |
| D294H | 0,042 | 0,019 | | 2,49 (1,67-3,71) |
| R142H | 0,013 | 0,008 | | 1,73 (0,92-3,28) |
| D84E | 0,0116 | 0,006 | | 2,10 (1,04-4,25) |

Les résultats ont montrés que la présence de variants RHC MC1R est fortement associée avec le risque de mélanome (P< 2.20 x 10⁻¹⁶). Le risque de mélanome augmente avec le nombre d'allèles RHC de OR = 2.29 avec un allèle variant, à OR =3.3 avec deux allèles variants. Par conséquent, l'effet de ces polymorphismes sur la maladie est presque cumulatif. Les résultats montrent les fréquences alléliques mineures pour les 5 polymorphismes RHC et, avec une exception (R142H), ils sont tous significativement, individuellement plus élevés chez les patients avec un mélanome que dans les contrôles.

De plus, l'association des variants de *MC1R* avec le mélanome reste significative après ajustement avec le type de peau (P =1.6 x 10⁻⁶), la couleur des cheveux (P = 9.3 x 10⁻⁷), la couleur des yeux (P = 6.48x 10⁻⁷), la couleur des cheveux (P = 8 x 10⁻⁷) et le nombre de naevus (P=3.3 x 10⁻⁸).

Les tableaux IIa et IIb résument les résultats pour les variants de *SLC45A2*.

**Tableau IIa : génotype SLC45A2 pour les sujets et les contrôles**

| SNPs | Génotype | Cas | Contrôles | P | OR (Cl) |
|---|---|---|---|---|---|
| SLC45A2 E272K | CC | 933 | 1,305 | | Référence |
| | CT | 33 | 119 | 1,10 x 10⁻⁶ | 0,39 (0,26-0,58) |
| | TT | 2 | 3 | | 0,93 (0,16-5,59) |
| MAF | | 0,019 | 0,044 | | |
| SLC45A2 L374F | GG | 895 | 1,160 | | référence |
| | GC | 65 | 246 | 2,12 x 10⁻¹⁵ | 0,34 (0,26-0,46) |
| | CC | 5 | 20 | | 0,32 (0,24-0,43) |
| MAF | | 0,39 | 0,10 | | |

| | | | | | |
|---|---|---|---|---|---|
| MAF : fréquence allélique mineure | | | | | |

**Tableau IIb : génotype SLC45A2 pour les sujets et les contrôles**

| **SLC45A2 haplotypes** | | **Cas (n=1882)** | **Contrôles (n=2794)** | **OR (Cl)** |
|---|---|---|---|---|
| 1 | C₈₁₄G₁₁₂₂ | 1807 (96) | 2509 (89,8) | référence |
| 2 | C₈₁₄C₁₁₂₂ | 39 (2,1) | 164 (5,9) | 0,33 (0,33-0,47) |
| 3 | T₈₁₄C₁₁₂₂ | 30 (1,16) | 112 (4) | 0,37 (0,25-0,56) |
| 4 | T₈₁₄G₁₁₂₂ | 6 (0,32) | 9 (0,34) | 0,92 (0,34-2,5) |

| **SLC45A2 diplotypes** | | **Cas (n=1882)** | **Contrôles (n=2794)** | **OR (Cl)** |
|---|---|---|---|---|
| 1/1 | CG/CG | 867 (92,1) | 1,126 (80,6) | Référence |
| ½ | CG/CC | 39 (4,1) | 148 (10,6) | 0,34 (0,24-0,49) |
| 1/3 | CG/TC | 28 (3) | 100 (7,16) | 0,36 (0,24-0,56) |

Les résultats montrent que les 2 variants de *SLC45A2* (L374F et E272K) sont étroitement associées avec le mélanome (P=2.12 x 10⁻¹⁵ et 1.10 x 10⁻⁶ respectivement), avec un déséquilibre de liaison presque complet entre les deux polymorphismes (D=0.036, D'=0.92 et r =0.59). La fréquence allélique SLC45A2 374 F dans les contrôles est de 0.90, alors qu'elle est de 0.96 pour les patients (P=1.30 10 x 10⁻¹¹). L'analyse de l'haplotype a confirmé l'association entre SLC45A2 et le mélanome (le test exact de FISHER appliqué aux fréquences d'Haplotype donne P=3.67 x 10⁻¹⁵, Tableau 2b). L'haplotype C₈₁₄G₁₁₂₂ est le plus fréquent, ayant été trouvé sur 96% des patients et 89.8% des contrôles, alors que les haplotypes C₈₁₄C₁₁₂₂ et T₈₁₄C₁₁₂₂ sont significativement plus fréquents dans les contrôles (une différence qui était due au variant L374F comme précisé dans le tableau 2b) et ont donc un effet protecteur contre le mélanome. Une analyse similaire des diplotypes indique que 2 diplotypes (CG/CC et CG/TC) qui sont présents jusqu'à 17.76 % des contrôles procure une protection contre le mélanome, alors que le diplotype le plus commun (CG/CG) est significativement plus fréquent pour les patients (92.1%) que pour les contrôles (80.1%) (La valeur de P du test exact de FISHERS des diplotypes = 1.89 x 10⁻¹³)

Finalement, comme les résultats ont montré que le gène *MATP*/*SLC45A2* est fortement lié avec le mélanome, la séquence codante entière (7 exons) de ce gène a été séquencée pour 48 patients atteints de mélanomes mais aucun autre variant non synonyme ou mutation pathogène du gène *SLC45A2* n'a été identifié.

De façon intéressante, l'association de *MATP*/*SLC45A2* L374F avec le mélanome reste significative après adaptation avec le type de peau (P = 1.6 x 10⁻⁶), la couleur des cheveux (P=8.7 x 10⁻⁷), la couleur des yeux (P = 1.21 x 10⁻⁴), et le nombre de naevus (P=1x10⁻⁵)_{.}

Dans le cas de *OCA2*, un seul SNP (OCA2-rs4778138) s'est avéré être faiblement associé avec le mélanome (P = 6.93 x 10⁻³). Les analyses des haplotypes et des diplotypes induit par les 3 SNPs de *OCA2* n'indiquent pas d'association significative plus importante de ce gène avec le mélanome, bien que l'haplotype TGT et le diplotype précédemment identifiés comme étant fortement associé avec la couleur des yeux clairs étaient plus fréquemment observé chez les patients atteints de mélanome que pour les contrôles. Cependant, l'association de ce SNP avec le mélanome n'était pas non plus significative après adaptation avec la couleur des yeux ou après correction pour des tests multiples.

Un modèle logistique incluant les facteurs génétiques, présentant une association statistique avec le mélanome (Variable MC1R, SLC45A2 , L374F, SLC45A2, E272 K et OCA2-rs4778138) a été développé. MC1R était pris en compte en fonction du nombre d'allèles RHC (0, 4 ou 2) et les trois autres étaient codés en combinant les génotypes les moins fréquents pour réduire le modèle génétique de deux paramètres sans perdre aucune information. Les associations des variants de *MC1R* et *SLC45A2* L374F avec le mélanome sont les seules à être maintenue, suggérant que ces facteurs génétiques jouent un rôle fort et indépendant dans la maladie.

Le tableau III montre le calcul combiné d'Ors des génotypes MC1R et SLC45A2 L374F.

**Tableau III : Calcul de l'OR combiné pour les génotypes MC1R et SLC45A L374F**

| Génotype MC1R | Génotype SLC45A2 | Cas | Contrôles | OR |
|---|---|---|---|---|
| 0 | 0 | 444 | 561 | Référence |
| 0 | 1 | 32 | 118 | 0,34 (0,23-0,51) |
| 0 | 2 | 3 | 13 | 0,29 (0,09-0,96) |
| 1 | 0 | 249 | 130 | 2,42 (1,89-3,09) |
| 1 | 1 | 20 | 30 | 0,84 (0,47-1,49) |
| 1 | 2 | 2 | 3 | 1,26 (0,22-7,19) |
| 2 | 0 | 39 | 11 | 4,48 (2,29-8,74) |
| 2 | 1 | 3 | 2 | 1,89 (0,38-1,52) |
| 2 | 2 | 0 | 0 | na |

La valeur 0 désigne les génotypes les plus fréquents qui sont pris comme référence (dans le cas de MC1R, l'absence de variants RHC, dans le cas de *SLC45A2* L374F, le génotype GG). 1 désigne la présence d'au moins un allèle RHC pour MC1R et du génotype CG pour *SLC45A2* L374F, et 2 désigne la présence de deux variants RHC pour MC1R et du génotype CC pour *SLC45A2* L374F.

« na » correspond à « non applicable ».

Le tableau III montre les ORs respectifs de toutes les combinaisons de génotypes des deux importants facteurs génétiques prédisposant au mélanome (MC1R et SLC45A2), et illustre les diverses combinaisons à risque ou protectrice.

Finalement, une étude des interactions potentielles entre la présence du variant de *MC1R* et de chaque variant de *SLC45A2* ou d'*OCA2* a été réalisée, mais aucune interaction gène-gène n'a pu être identifiée.

### Association des facteurs cliniques avec le mélanome

Les caractéristiques cliniques principales de la cohorte mélanome et 220 contrôles ont été étudiés. Cette étude a mis en évidence que les facteurs de risque les plus importants correspondaient à un nombre de naevus > (ou égale) à 50 (OR=5.91), à une couleur des yeux claire (OR =2.55), au type de peau I ou II (OR=2.35), à une couleur des cheveux claire (OR=2.18) et à des lentigines solaires (OR=3.19)

### Association des facteurs génétiques avec les facteurs cliniques

Dans la mesure où ces trois gènes ont été identifiés comme agissant tous les trois sur la pigmentation, des analyses d'association individuelle des facteurs génétiques sur les facteurs principaux de risque clinique ont aussi été réalisées et adaptées pour permettre de distinguer le statut contrôle de celui de patient atteint de mélanome.

La plus forte association était celle entre OCA2 et la couleur des yeux (P<2.20 x 10⁻¹⁶ pour chacun des trois SNPs OCA2), ce qui confirme l'association récemment démontrée entre ces SNPs et la couleur des yeux (DUFFY et al., Am. J. Hum. Genet., vol.80, p:241-52, 2007). Nous avons aussi testé l'association spécifique de l'haplotype TGT avec la couleur des yeux, et avons aussi trouvé une valeur de P très significative (P<2.20 x 10⁻¹⁶). Les trois variants d'*OCA2* et l'haplotype TGT étaient aussi fortement associés avec la couleur des cheveux (P< 0.0001)

Le SNP *SLC45A2* L374F s'est révélé étroitement associé à la couleur des yeux (lP=2.09 x 10⁻⁵), à la couleur des cheveux (P=6.96 x 10⁻⁸) et au type de peau (P=6.2 x 10⁻⁸).

Finalement, les variants de *MC1R* ont montré une association significative avec la couleur des cheveux (P= 1.49 x 10⁻⁸) et avec le type de peau (P =1.30 x 10⁻⁸)

Pour résumer, le type de peau est fortement associé avec les variants de *SLC45A2* et de *MC1R*, la couleur des yeux avec *OCA2* et dans une moindre importance avec *SLC45A2*, et la couleur des cheveux avec les trois gènes.

Remarquablement, le nombre de naevus, qui était l'indice clinique du mélanome le plus important dans notre étude, n'était statistiquement pas associé à l'un de ces gènes de pigmentation.

### Intégration de la génétique et des facteurs cliniques

Sur la base des associations les plus fortes identifiées dans cette étude entre les facteurs cliniques et génétiques et le mélanome, nous avons développé un modèle logique pour tester la persistance des associations avec le mélanome et nous avons recalculé les ORs correspondants quand les facteurs de risque étaient combinés.

Dans ce modèle, seul le nombre de naevus a été inclus car il s'agit du seul facteur de risque clinique qui ne montrait aucune association avec chacun des 3 gènes. Nous avons observé que l'association des variants de *MC1R* et *SLC45A2* L374F avec le mélanome était encore hautement significative en présence d'un nombre de naevus (P =1.14x10⁻⁷ et 3.32 x 10⁻⁶ respectivement)

De plus, il était facile de calculer les ORs cumulatifs résultant de la combinaison de ces facteurs. Dans ce modèle, l'OR des personnes ayant un nombre de naevus > (ou égale) à 50, un allèle RHC pour MC1R et le génotype le plus fréquent SLC45A2 L374 F (GG), OR =5.26 x 3.85 =20.25, donne une approximation du risque du mélanome pour ces personnes qui est 20 fois plus élevé que celles ayant un nombre de naevus <50, pas d'allèle RHC, et un des génotypes SLC45A2 protecteurs.

Finalement, la présente étude a permis de déterminer que le génotype GG SLC45A2 L374 F constitue un nouveau paramètre important permettant d'apprécier le risque de mélanome chez un patient, l'allèle SLC45A2 374F étant clairement associé avec le risque de mélanome, alors que l'allèle SLC45A2 374L protégerait lui du mélanome.

En outre, le fait que l'effet de ce variant de *SLC45A2* persiste après stratification sur les caractéristiques de pigmentation ou dans un modèle de régression logique intégrant les 2 facteurs de risque génétique et le nombre de naevus suggère qu'au regard du risque de mélanome, l'information liée au génotype SLC45A2 L374F n'est pas redondante avec les caractéristiques de pigmentation et que ce variant est donc un facteur de risque de mélanome fort et indépendant.

Les résultats concernant le marqueur SLC45A2 L374F ont été par la suite confirmés sur une cohorte d'individus espagnols (Fernandez LP et al, Human mutation 0,1-7, 2008).

D'autre part, les présents inventeurs ont également confirmé les résultats portant sur les marqueurs SLC45A L374F et MC1R sur une population italienne.

Ces résultats prouvent donc sans ambigüité que les marqueurs SLC45A2 L374F et également MC1R RHC sont des marqueurs fiables de prédisposition au mélanome, qui peuvent être utilisés seuls ou en association pour évaluer très efficacement le risque de prédisposition au mélanome chez l'homme en général.

## Revendications

1. Un procédé *in vitro* destiné à identifier un sujet atteint ou présentant une prédisposition au cancer de la peau, **caractérisé en ce qu'**il comprend l'étape d'analyse d'un échantillon biologique provenant dudit sujet par :
a) la détection d'un polymorphisme du gène *MATP*/*SLC45A2-*, de SEQ ID NO : 1, associé à un seul nucléotide (SNP) est choisi dans le groupe comprenant le SNP rs16891982, correspondant au nucléotide N à la position 301 de SEQ ID NO : 2, le nucléotide G étant associé à un cancer cutané et aboutissant à la présence d'une phénylalanine en position 374 de la protéine MATP/SLC45A2 SEQ ID NO :3 et le SNP rs26722, correspondant au nucléotide N à la position 301 de SEQ ID n° 4, le nucléotide C étant associé à un cancer cutané et aboutissant à la présence d'un résidu Glutamate en position 272 de la protéine MATP/SLC45A2 de SEQ ID NO :3.

2. Le procédé selon la revendication 1, comprenant en outre l'étape b) de détection d'un polymorphisme du gène *MC1R*, de SEQ ID NO : 10.

3. Le procédé selon l'une quelconque des revendications 1 ou 2, **caractérisé en ce que** ledit sujet est un mammifère, de préférence un humain.

4. Le procédé selon l'une quelconque des revendications 1 à 3, **caractérisé en ce que** ledit cancer de la peau est un mélanome.

5. Le procédé selon l'une quelconque des revendications précédentes, **caractérisé en ce que** ledit polymorphisme du gène *MATP*/*SLC45A2* associé à un seul nucléotide (SNP) correspond au SNP rs16891982 correspondant au nucléotide N à la position 301 de SEQ ID NO : 2, le nucléotide G étant associé à un cancer cutané et aboutissant à la présence d'une phénylalanine en position 374 de la protéine MATP/SLC45A2 de SEQ ID NO :3.

6. Le procédé selon l'une quelconque des revendications 2 à 5, **caractérisé en ce que** ledit polymorphisme du gène *MC1R* associé à une prédisposition au cancer de la peau correspond à un polymorphisme associé à un seul nucléotide (SNP) choisi dans le groupe comprenant le SNP rs1805006 correspondant au nucléotide N à la position 26 de SEQ ID NO 6, le nucléotide A étant associé à un cancer cutané et aboutissant à la présence d'un Glutamate en position 84 de la protéine MC1R de SEQ ID NO :5, le SNP rs1805007 correspondant au nucléotide N à la position 301 de SEQ ID NO 7, le nucléotide T étant associé à un cancer cutané et aboutissant à la présence d'une cystéine en position 151 de la protéine MC1R de SEQ ID NO :5, le SNP rs1805008 correspondant au nucléotide N à la position 301 de SEQ ID NO 8, le nucléotide T étant associé à un cancer cutané et aboutissant à la présence d'un tryptophane en position 160 de la protéine MC1R de SEQ ID NO :5, le SNP rs1805009 correspondant au nucléotide N à la position 26 de SEQ ID NO 9, le nucléotide C étant associé à un cancer cutané et aboutissant à la présence d'une histidine en position 294 de la protéine MC1R de SEQ ID NO :5.

7. Le procédé selon l'une quelconque des revendications précédentes, comprenant en outre une étape d'analyse de l'expression du gène *MATPlSLC45A2* dans ledit échantillon biologique.

8. Le procédé selon la revendication 7, **caractérisé en ce qu'**il comprend en outre une étape de comparaison du niveau d'expression du gène *MATP*/*SLC45A2* dans ledit échantillon biologique avec le niveau d'expression du gène *MATPlSLC45A2* dans un échantillon contrôle.

## Patentansprüche

1. *In vitro*-Verfahren, das dazu bestimmt ist, ein Individuum, das an Hautkrebs leidet oder eine Veranlagung dafür aufweist, zu identifizieren, **dadurch gekennzeichnet, dass** es den Schritt einer Analyse einer von dem Individuum stammenden biologischen Probe durch:
a) den Nachweis eines Polymorphismus des Gens *MATPlSLC45A2* der SEQ ID NO: 1, der mit einem einzigen Nukleotid assoziiert ist (SNP), ausgewählt in der Gruppe umfassend den SNP r16891982 entsprechend dem Nukleotid N an der Position 301 von SEQ ID NO: 2, wobei das Nukleotid G mit einem Hautkrebs assoziiert ist und zu dem Vorhandensein eines Phenylalanins an Position 374 des Proteins MATP/SLC45A2 SEQ ID NO: 3 führt, und den SNP rs26722 entsprechend dem Nukleotid N an der Position 301 von SEQ ID NO: 4, wobei das Nukleotid C mit einem Hautkrebs assoziiert ist und zu dem Vorhandensein eines Glutamat-Rests an Position 272 des Proteins MATP/SLC45A2 der SEQ ID NO: 3 führt,
umfasst.

2. Verfahren nach Anspruch 1, welches außerdem den Schritt b) eines Nachweises eines Polymorphismus des Gens *MC1R* der SEQ ID NO: 10 umfasst.

3. Verfahren nach einem der Ansprüche 1 oder 2, **dadurch gekennzeichnet, dass** das Individuum ein Säugetier, vorzugsweise ein Mensch ist.

4. Verfahren nach einem der Ansprüche 1 bis 3, **dadurch gekennzeichnet, dass** der Hautkrebs ein Melanom ist.

5. Verfahren nach einem der vorangegangenen Ansprüche, **dadurch gekennzeichnet, dass** der Polymorphismus des Gens *MATP*/*SLC45A2*, der mit einem einzigen Nukleotid assoziiert ist (SNP), dem SNP rs16891982 entsprechend dem Nukleotid N an der Position 301 von SEQ ID NO: 2, wobei das Nukleotid G mit einem Hautkrebs assoziiert ist und zu dem Vorhandensein eines Phenylalanins an Position 374 des Proteins MATP/SLC45A2 der SEQ ID NO: 3 führt, entspricht.

6. Verfahren nach einem der Ansprüche 2 bis 5, **dadurch gekennzeichnet, dass** der Polymorphismus des Gens *MC1R*, der mit einer Veranlagung für Hautkrebs assoziiert ist, einem mit einem einzigen Nukleotid assoziierten Polymorphismus (SNP), ausgewählt in der Gruppe, umfassend den SNP rs1805006 entsprechend dem Nukleotid N an Position 26 von SEQ ID NO: 6, wobei das Nukleotid A mit einem Hautkrebs assoziiert ist und zu dem Vorhandensein eines Glutamats an Position 84 des Proteins MC1 R der SEQ ID NO: 5 führt, den SNP rs1805007 entsprechend dem Nukleotid N an Position 301 von SEQ ID NO: 7, wobei das Nukleotid T mit einem Hautkrebs assoziiert ist und zu dem Vorhandensein eines Cysteins an Position 151 des Proteins MC1 R der SEQ ID NO: 5 führt, den SNP rs1805008 entsprechend dem Nukleotid N an Position 301 von SEQ ID NO: 8, wobei das Nukleotid T mit einem Hautkrebs assoziiert ist und zu dem Vorhandensein eines Tryptophans an Position 160 des Proteins MC1R der SEQ ID NO: 5 führt, den SNP rs1805009 entsprechend dem Nukleotid N an Position 26 von SEQ ID NO: 9, wobei das Nukleotid C mit einem Hautkrebs assoziiert ist und zu dem Vorhandensein eines Histidins an Position 294 des Proteins MC1 R der SEQ ID NO: 5 führt, entspricht.

7. Verfahren nach einem der vorangegangenen Ansprüche, welches außerdem einen Schritt einer Analyse der Expression des Gens *MATPlSLC45A2* in der biologischen Probe umfasst.

8. Verfahren nach Anspruch 7, **dadurch gekennzeichnet, dass** es außerdem einen Schritt eines Vergleichs des Expressionsniveaus des Gens *MATPlSLC45A2* in der biologischen Probe mit dem Expressionsniveau des Gens *MATP*/*SLC45A2* in einer Kontrollprobe umfasst.

## Claims

1. *In vitro* method intended to identify a subject suffering from or having a predisposition to skin cancer, **characterised in that** it comprises the step for analysing a biological sample from said subject by:
a) detecting a polymorphism of the *MATP*/*SLC45A2* gene of SEQ ID NO:1, associated with a single nucleotide (SNP) chosen in the group comprising SNP rs16891982 comprising an N nucleotide in position 301 of SEQ ID NO:2, the G nucleotide being associated with skin cancer and resulting in the presence of a phenylalanine in position 374 of the protein MATP/SLC45A2 of SEQ ID NO:3 and SNP rs26722, corresponding to an N nucleotide in position 301 of SEQ ID NO:4, the C nucleotide being associated with skin cancer and resulting in the presence of a glutamate residue in position 272 of the MATP/SLC45A2 protein of SEQ ID NO:3.

2. Method according to claim 1, further comprising the step b) for detecting a polymorphism of the *MC1R* gene of SEQ ID NO:10.

3. Method according to any of claims 1 or 2, **characterised in that** said subject is a mammal, preferably a human.

4. Method according to any of claims 1 to 3, **characterised in that** said skin cancer is a melanoma.

5. Method according to any of the above claims, **characterised in that** said polymorphism of the *MATP*/*SLC45A2* gene associated with a single nucleotide (SNP) corresponds to the SNP rs16891982 comprising an N nucleotide in position 301 of SEQ ID NO:2, the G nucleotide being associated with skin cancer and resulting in the presence of a phenylalanine in position 374 of the protein MATP/SLC45A2 of SEQ ID NO:3.

6. Method according to any of claims 2 to 5, **characterised in that** said polymorphism of the *MC1R* gene associated with predisposition to skin cancer corresponds to a polymorphism associated with a single nucleotide (SNP) chosen in the group comprising SNP rs1805006 corresponding to an N nucleotide in position 26 of SEQ ID NO:6, the A nucleotide being associated with skin cancer and resulting in the presence of a glutamate in position 84 of the MC1R protein of SEQ ID NO:5, the SNP rs1805007 corresponding to an N nucleotide in position 301 of SEQ ID NO:7, the T nucleotide being associated with skin cancer and resulting in the presence of a cysteine in position 151 of the MC1R protein of SEQ ID NO:5, the SNP rs1805008 corresponding to an N nucleotide in position 301 of SEQ ID NO:8, the T nucleotide being associated with skin cancer and resulting in the presence of a tryptophan in position 160 of the MC1R protein of SEQ ID NO:5, the SNP rs1805009 corresponding to an N nucleotide in position 26 of SEQ ID NO:9, the C nucleotide being associated with skin cancer and resulting in the presence of a histidine in position 294 of the MC1R protein of SEQ ID NO:5.

7. Method according to any of the above claims, further comprising a step of analysing the expression of the *MATP*/*SLC45A2* gene in said biological sample.

8. Method according to claim 7, **characterised in that** it further comprises a step for comparing the expression level of the *MATP*/*SLC45A2* gene in said biological sample with the expression level of the *MATP*/*SLC45A2* gene in a control sample.
